# EUROPEAN PATENT SPECIFICATION

(11) **EP 3 670 645 B1**
(45) Date of publication and mention of the grant of the patent: **24.06.2026**
(21) Application number: 19216745.0
(22) Date of filing: 16.12.2019
(51) Int. Cl.: C12N 1/04, C12N 1/20

(54) **REAGENT FOR PROTECTION OF MICROORGANISMS DURING LYOPHILIZATION PROCESS**
REAGENZ ZUM SCHUTZ VON MIKROORGANISMEN WÄHREND EINES LYOPHILISIERUNGSPROZESSES
RÉACTIF POUR LA PROTECTION DE MICRO-ORGANISMES PENDANT LE PROCESSUS DE LYOPHILISATION

(30) Priority: 17.12.2018 PL 42821518
(43) Date of publication of application: 24.06.2020
(73) Proprietor: Uniwersytet Gdanski, 80-309 Gdansk (PL)
(72) Inventor: Czajkowski, Robert, 80-382 Gdansk (PL); Dorota, Krzyzanowska, 81-572 Gdynia (PL); Tomasz, Maciag, 85-337 Bydgoszcz (PL); Sylwia, Jafra, 80-382 Gdansk (PL); Siwinska, Joanna, 80-177 Gdansk (PL)
(74) Representative: Czarnik, Maciej

(56) References cited:
- CN-A- 106 635 802
- MACIAG TOMASZ ET AL: "The Great Five-an artificial bacterial consortium with antagonistic activity towards Pectobacterium spp. and Dickeya spp.: formulation, shelf life, and the ability to prevent soft rot of potato in storage", APPLIED MICROBIOLOGY AND BIOTECHNOLOGY, vol. 104, no. 10, 26 March 2020 (2020-03-26), pages 4547 - 4561, XP037107818, ISSN: 0175-7598, [retrieved on 20200326], DOI: 10.1007/S00253-020-10550-X
- "ATCC BACTERIAL CULTURE GUIDE", 2015, pages 1 - 36, XP055686675, Retrieved from the Internet <URL:https://www.atcc.org/~/media/PDFs/Culture%20Guides/ATCC_Bacterial_Culture_Guide.ashx> [retrieved on 20200417]
- VEKEMAN B. ET AL: "A generally applicable cryopreservation method for nitrite-oxidizing bacteria", SYSTEMATIC AND APPLIED MICROBIOLOGY, vol. 36, no. 8, 1 December 2013 (2013-12-01), AMSTERDAM, NL, pages 579 - 584, XP055686800, ISSN: 0723-2020, DOI: 10.1016/j.syapm.2013.07.002
- GEORGE F. ET AL: "Freezing of in vitro produced bovine embryos in animal protein-free medium containing vegetal peptones", THERIOGENOLOGY, vol. 66, no. 5, 15 September 2006 (2006-09-15), pages 1381 - 1390, XP027930287, ISSN: 0093-691X, [retrieved on 20060915]

## Description

The invention relates to a reagent developed specifically to protect bacteria during lyophilization (freeze-drying).. The reagent may be used particularly in the production of probiotics.

Lyophilization is a process involving the evaporation of solvent from frozen substances - the process called freeze drying. Lyophilization is a method often used in case of bacteria to assure long-term preservation of cell viability in preparations of commercial probiotics, as well as for the storage of microorganisms with defined characteristics and properties in collections of microorganisms including bacteria.

An important problem to be solved in the lyophilization process is that the procedure decreases bacterial viability and survival both because of freezing itself but also as a result of changes in the shape of bacterial cells due to the loss of water. In collections of microorganisms, such as the international ATCC collection (American Type Culture Collection) located in the United States (USA), a dedicated reagent is routinely used to protect bacterial cells in the lyophilization process. This reagent, hereinafter referred to as "Reagent 18", contains (for each 100 mL of reagent volume): 0.75 g of Tryptone Soya Broth (TSB), 10 g sucrose, 5 g bovine serum albumin (BSA). The Reagent 18 is effective, but it contains expensive and animal-derived compound: bovine serum albumin (bovine serum).

Existing patent EP2167639 describes the use of plant peptone, including wheat peptone, for preparation of bacterial media. Wheat peptone, in the described patent is, however, a source of non-animal protein nitrogen, and is not used in the invention to protect microorganisms against environmental conditions, including ones during the lyophilization process (freeze-drying).

The invention relates to the development of an effective means of protecting bacteria ensuring high survival of bacteria during freeze-drying.

The reagent for protecting bacteria during lyophilization according to the invention contains tryptone-soy broth (TSB), sucrose and wheat peptone.

Preferably, the reagent contains from 0.1 to 1.1% w / v (weight / volume) wheat peptone, preferably ranges from 0.1-0.5% by weight wheat peptone and even more preferably 0.2-0.3% by weight wheat peptone.

Preferably, the reagent contains 0.5 to 1% w / v TSB, 10 w / v sucrose, 0.1 to 1.1% w / v wheat peptone, or the preferred above ranges for peptone, and in addition, in the form of a liquid, it contains a medium (solvent), especially in the form of water.

Preferably the reagent contains ca. 0.7% by weight TSB +/- 0.3%, about 30% by weight sucrose +/- 5 and 0.2-0.3% by weight wheat peptone.

Preferably, the reagent consists of TSB, sucrose, wheat peptone and, in the form of a liquid solution, a medium (solvent), especially water.

The invention also relates to the use of the reagent described above for the protection of bacterial probiotic strains during lyophilization.

In the reagent according to the invention, described as "Reagent PS", wheat peptone is used in a concentration from 0.1 to 1.1% w / v, in particular 0.127 to 1.02% w / v.

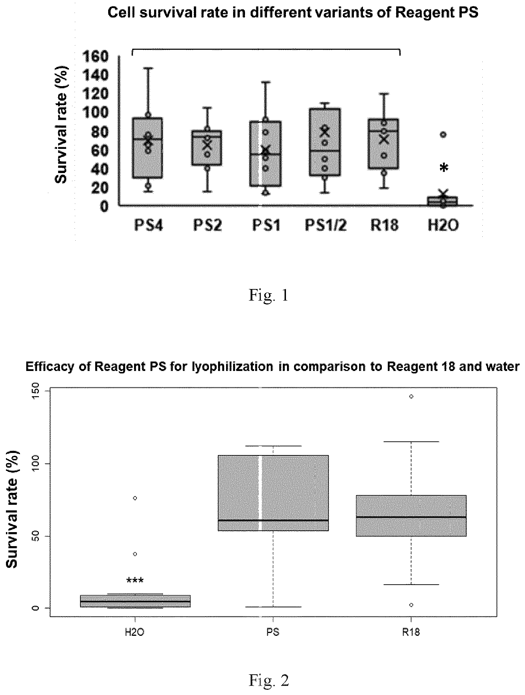

The most favorable range of this component (wheat peptone) has also been developed. Due to this concentration, the reagent very efficiently protects bacterial viability during lyophilization. A positive effect of Reagent PS on the survival of bacteria has been demonstrated. The invention reduces the cost of lyophilization, as well as it ensures high efficiency of freeze-drying. The invention has been described in Examples (Examples 1-5) and its effectiveness is additionally illustrated in Figure 1 and Figure 2 presenting the box plot graphs show survival of bacteria described in more detail in Example 5.

The invention is set out in the appended set of claims, which determine the scope of the invention.

### Example 1

### The method to obtain Reagent PS

The lyophilization reagent (Reagent PS) with the developed composition for each 100 mL reagent volume: 0.75g TSB, 10g sucrose and 0.255g wheat peptone is prepared by dissolving all the ingredients described above in demineralized water (solvent). The Reagent PS is sterilized at 121 °C at 0.7 Atm for 15 minutes. After sterilization and cooling to room temperature, the reagent is ready to use.

### Example 2

The reagent is prepared as described in Example 1 except 0.127 g of peptone is used.

### Example 3

The reagent is prepared as described in Example 1 except 0.510 g peptone is used.

### Example 4

The reagent is prepared as described in Example 1, except that 1.020 g of peptone is used.

### Example 5

Analyses of the effectiveness of the reagent containing different concentrations of peptone.

The effectiveness of the reagents obtained as described in Examples 1-4 above ("Reagent PS") was tested by comparing the survival of 4 bacterial strains, lyophilized using, as a cryoprotectant/lyoprotectant, either reagent "Reagent PS", reagent "Reagent 18" (recommended by ATCC) or water (control without protective lyoprotectant). Versions of the "Reagent PS" were named based on the concentration of wheat peptone: PS4 (1.020g / 100ml); PS2 (0.510g / 100ml); PS1 (0.255g / 100ml); PS1 / 2 (0.127g / 100ml). The bacterial strains used for the analyses included strains with potential biological control activity, namely *Serratia plymuthica* A294, *Serratia rubidea* H440, *Pseudomonas fluorescens* CHAO, and a probiotic strain administrated orally in humans: *Lactobacillus rhamnosus* GG.

Bacteria were grown overnight (16h) in liquid media. In the case of probiotic bacteria, the medium was De Man, Rogosa and Sharpe medium (MRS). In other cases, Trypton Soy Broth (TSB) was used. Cultivation was carried out at 28 or 37 °C depending on the growth requirements of individual strain. The bacteria were pelleted by centrifugation (6500 rcf, 5 min) and suspended in the abovementioned lyophilization reagents or in water (control). Bacterial titer in pre-lyophilization suspensions was determined (in colony-forming units per milliliter, CFU / mL) by plating serial dilutions on suitable agar solid growth media and counting the growing bacterial colonies. The suspensions were frozen overnight at -80 °C and then lyophilized at -50 °C for 24h. The obtained lyophilizates were dissolved in sterile distilled water in a volume equal to the initial volume of the suspensions and the number of viable cells in each sample was determined using the method described above. The titer of viable (alive) cells in lyophilizates was compared to the initial bacterial titer in pre-lyophilization suspensions to determine the percentage of surviving cells (survival rate). For the 4 bacterial strains tested, no significant differences were found in the survival of lyophilization in the reagents prepared according to Example 1-4 and lyophilized in Reagent 18 (R18). Survival was significantly higher than the survival of lyophilization in water (Kruskal-Wallis chi2 = 15.341, df = 6, p = 0.01776; differences between groups were checked by Dunn's test).

The survival results are shown in Figure 1, where the following samples were compared:
PS4 - lyophilization in "Reagent PS" prepared according to example 4,
PS2 - lyophilization in "Reagent PS" prepared according to example 3,
PS1 - lyophilization in "Reagent PS" prepared according to example 1,
PS1 / 2 - lyophilization in "Reagent PS" prepared according to example 2,
R18 - lyophilization in "Reagent 18",
H2O - freeze drying in water

The survival of tested bacterial strains following the lyophilization process was determined by calculating the ratio of the cfu in the lyophilizate to the cfu of the initial bacterial suspension. The survival rate following lyophilization in water was significantly lower than in the "Reagent 18" (p = 0.00095). Moreover, there were no significant differences in bacterial survival between the "Reagent 18" and the "Reagent PS" in any of the tested versions: PS4 (1.02g peptone / 100mL), PS2 (0.510g peptone / 100mL), PS1 (0.255g peptone / 100mL), and PS1 / 2 (0.127g peptone / 100mL). In the box plot graph, the boxes indicate the inter-quartile range Q2-Q3, the bold line indicates the median, the whisker indicates standard deviations, and the dots show outliers. Asterisks indicate results that are significantly different from "Reagent 18" ("*" p <0.05). For some tested strains, the survival rate slightly exceeded 100% due to a method error (resulting from the necessity to sample a thick bacterial suspension). Comparisons of the effect of tested reagents on cell survival were performed using data pooled for all strains tested *(Serratia plymuthica* A294, *Serratia rubidea* H440, *Pseudomonas fluorescens* CHAO, *Lactobacillus rhamnosus* GG).

Testing the effectiveness of the reagent with a peptone concentration equal to 0.255g wheat peptone / 100mL.

The effectiveness of the "Reagent PS" obtained as described in Example 1 was tested by comparing the survival rate of 15 strains of microorganisms lyophilized using, as a cryoprotectant/lyoprotectant, either "Reagent PS", "Reagent 18" (recommended by ATCC), or water (control, without protective cryoprotectant/lyoprotectant). The microbial strains included a model organism, namely: 1) bacterial starins: *Escherichia coli* DH5α, strains with potential for biological control of pathogens: *Serratia plymuthica* A294, *Enterobacter amnigenus* A167, *Rahnella aquatilis* H145, *Serratia rubidaea* H440, *Serratia rubidaea* H469, *Pseudomonas fluorescens* Pf-5, *Ochrobactrum* sp. A44, *Bacillus subtilis* 168, *Pseudomonas donghuensis* P482, oral probiotic strains: *Bacillus coagulans, Lactobacillus rhamnosus* GG, *Lactobacillus brevis, Lactobacillus rhamnosus* 573 and yeast *Saccharomyces boulardi* for comparison of the results to the bacterial strains,. The microbial survival rate following lyophilization was determined as described above in section describing the assessment of efficacy of "Reagent PS" containing different concentrations of peptone.

For 13 of the 15 tested strains, the effectiveness of the "Reagent PS" and "Reagent 18" was similar, i.e. it provided 38% to 100% survival for "Reagent PS" and 40% to 100% for "Reagent 18", depending on the microbial strain. At the same time, this survival rate with lyoprotectants was ca. 3.5 to 4 times higher compared to the survival of strains lyophilized in water (control) (Table 1). Only for 2 of the 15 strains: *Escherichia coli* DH5α and *Ochrobactrum* sp. A44, the survival rate was relatively high in distilled water (control) and was 76% and 37%, respectively (Table 1). Lyophilization of these bacteria probably does not require the use of a lyoprotectant to improve the survival rate of bacterial strains. In contrast, the survival rate of a single of the 16 strains, *Saccharomyces boulardi,* was low even when lyopreservation reagents were applied - the highest survival was obtained by using the Reagent 18 and it was only 2.38% (Table 1). In this case, obtaining a higher survival rate would probably require the use of a lyoprotectant with an individually tailored composition. For all bacterial strains tested, the protective effect of applying "Reagent PS" according to the invention has been confirmed.

**Table 1. Survival rate of the lyophilization process by bacterial strains and yeast strain lyophilized using two different lyopreservation reagents and water (control). Survival rate was determined according to the formula: survival rate = (CFU / mL in lyophilizates × 100% / CFU / mL in pre-lyophilization suspensions). If provided, the information in brackets under the heading 'Strain' refers to the name of the commercial preparation from which the microorganisms - bacteria or yeast, was derived.**

| | | Survival rate during lyophilization (%) | | |
|---|---|---|---|---|
| No. | Strain | H₂O | Reagent PS | Reagent 18 |
| 1 | *Serratia plymuthica* A294 | 8,14% | 111,65% | 61,23% |
| 2 | *Enterobacter amnigenus* A167 | 5,23% | 50,16% | 65,09% |
| 3 | *Rahnella aquatilis* H145 | 10,10% | 57,00% | 65,37% |
| 4 | *Serratia rubidaea* H440 | 9,34% | 112,03% | 58,06% |
| 5 | *Serratia rubidaea* H469 | 2,06% | 61,44% | 115,13% |
| 6 | *Pseudomonas fluorescens* CHAO | 1,18% | 59,90% | 55,21% |
| 7 | *Pseudomonas fluorescens* Pf-5 | 0,38% | 61,86% | 146,29% |
| 8 | *Ochrobactrum* sp. A44 | 37,43% | 59,36% | 88,96% |
| 9 | *Bacillus subtilis 168* | 4,80% | 75,49% | 83,61% |
| 10 | *Pseudomonas donghuensis* P482 | 0,05% | 37,92% | 40,99% |
| 11 | *Escherichia coli* DH5α | 76,06% | 12,93% | 16,28% |
| 12 | *Saccharomyces boulardi* (ENTEROL 250) | 0,04% | 0,95% | 2,38% |
| 13 | *Bacillus coagulans* (Colinox) | 4,49% | 56,76% | 44,80% |
| 14 | *Lactobacillus rhamnosus* GG (Dicoflor 50) | 3,00% | 111,25% | 64,62% |
| 15 | *Lactobacillus brevis* | 5,00% | 100,00% | 72,22% |
| 16 | *Lactobacillus rhamnosus* 573 (Lactovaginal) | 0,53% | 110,71% | 60,00% |

In order to test whether there are significant differences in the effectiveness of the "Reagent 18" and the newly developed "Reagent PS", the survival of the 15 lyophilized strains in 2 different lyoprotectant reagents and in water was compared using a statistical test for 3 independent samples. Based on the calculations made, it was observed that the microbial survival rate following lyophilization in water (control) was the lowest and significantly different (Kruskal-Wallis chi2 = 23.678; df = 2, p = 7.218e-06) from the survival rate in "Reagent 18" (Dunn test p < 0.0001) and in "Reagent PS" (p < 0.0001). The results pooled for all 15 strains tested are shown in Figure 2.

The lyophilization survival rate for the tested strains was determined by calculating the ratio of the CFU in the lyophilizate to the CFU in the bacterial suspension prior the lyophilization procedure. The lyophilization survival rate in water was significantly lower than in the reagent according to the invention. There were no significant differences in the survival rate between the "Reagent 18" and the "Reagent PS"., Considering "Reagent 18" is recognized worldwide as effective, these results confirm the significant effectiveness of the newly-developed "Reagent PS" according to the invention because. In the box plot shown in Figure 2, the box indicates the inter-quartile range Q2-Q3, the bold line indicates the median, the whisker indicates standard deviations, and the dots indicate outliers. Asterisks mark results with statistically significant differences (*** "p <0.001). In the graph, H2O stands for freeze-drying in water, PS for freeze-drying in "Reagent PS", and R18 for freeze-drying in "Reagent 18". For some strains, the survival rate slightly exceeds 100% due to a method error (the need to take a sample from a thick bacterial suspension).

## Claims

1. Reagent for protecting bacteria during lyophilization, **characterized by** the fact that it contains tryptone soy broth (TSB), sucrose and wheat peptone.

2. Reagent according to the claim 1, **characterized by** the fact that it contains from 0.1 to 1.1% w / v (weight / volume) wheat peptone.

3. The reagent according to the claim 2 **characterized by** the fact that it contains from 0.1-0.5% by weight of wheat peptone.

4. The reagent according to the claim 3 **characterized by** the fact that it contains from 0.2-0.3% by weight of wheat peptone.

5. The reagent according to the claims 1-4, **characterized by** the fact that it contains from 0.5 to 1% w / v (weight / volume) TSB, 10% w / v of sucrose, and medium.

6. The reagent according to the claims 1-5 **characterized by** the fact that the reagent consists of TSB, sucrose, wheat peptone and medium.

7. The reagent according to the claim 5 or 6 **characterized by** the fact that the medium (solvent) is water.

8. The use of a reagent according to any of the claims 1-7 to protect bacterial probiotic strains during lyophilization.

## Patentansprüche

1. Reagenz zum Schutz von Bakterien während der Lyophilisation, **dadurch gekennzeichnet, dass** es Tryptone-Soja-Brühe (TSB), Saccharose und Weizenpepton enthält.

2. Reagenz nach Anspruch 1, **dadurch gekennzeichnet, dass** es 0,1 bis 1,1 % m/v (Gewicht/Volumen) Weizenpepton enthält.

3. Reagenz nach Anspruch 2, **dadurch gekennzeichnet, dass** es 0,1 bis 0,5 Gew.-% Weizenpepton enthält.

4. Reagenz nach Anspruch 3, **dadurch gekennzeichnet, dass** es 0,2 bis 0,3 Gew.-% Weizenpepton enthält.

5. Reagenz nach den Ansprüchen 1 bis 4, **dadurch gekennzeichnet, dass** es 0,5 bis 1 % m/v (Gewicht/Volumen) TSB, 10 % m/v Saccharose und ein Medium enthält.

6. Reagenz nach den Ansprüchen 1 bis 5, **dadurch gekennzeichnet, dass** das Reagenz aus TSB, Saccharose, Weizenpepton und einem Medium besteht.

7. Reagenz nach Anspruch 5 oder 6, **dadurch gekennzeichnet, dass** das Medium (Lösungsmittel) Wasser ist.

8. Verwendung eines Reagenzes nach einem der Ansprüche 1 bis 7 zum Schutz probiotischer Bakterienstämme während der Lyophilisation.

## Revendications

1. Réactif destiné à protéger des bactéries lors de la lyophilisation, **caractérisé en ce qu'**il contient du bouillon tryptone-soja (TSB), du saccharose et de la peptone de blé.

2. Réactif selon la revendication 1, **caractérisé en ce qu'**il contient de 0,1 à 1,1 % m/v (poids/volume) de peptone de blé.

3. Réactif selon la revendication 2, **caractérisé en ce qu'**il contient de 0,1 à 0,5 % en poids de peptone de blé.

4. Réactif selon la revendication 3, **caractérisé en ce qu'**il contient de 0,2 à 0,3 % en poids de peptone de blé.

5. Réactif selon les revendications 1 à 4, **caractérisé en ce qu'**il contient de 0,5 à 1 % m/v (poids/volume) de TSB, 10 % m/v de saccharose et un milieu.

6. Réactif selon les revendications 1 à 5, **caractérisé en ce que** le réactif est constitué de TSB, de saccharose, de peptone de blé et d'un milieu.

7. Réactif selon la revendication 5 ou 6, **caractérisé en ce que** le milieu (solvant) est de l'eau.

8. Utilisation d'un réactif selon l'une quelconque des revendications 1 à 7 pour protéger des souches bactériennes probiotiques lors de la lyophilisation.
